(19)

**Europäisches Patentamt**
**European Patent Office**
**Office européen des brevets**

(11)  **EP 3 153 854 B1**

(12)  **EUROPEAN PATENT SPECIFICATION**

(45) Date of publication and mention
of the grant of the patent:
**31.03.2021  Bulletin 2021/13**

(51) Int Cl.:
*G01N 33/22* *(2006.01)*    *G01N 25/18* *(2006.01)*
*G01F 1/68* *(2006.01)*

(21) Application number: **15188323.8**

(22) Date of filing: **05.10.2015**

(54)  **DETERMINATION OF VOLUMETRIC FLOW RATE OF A GAS IN A GAS FLOW**

BESTIMMUNG VON VOLUMENDURCHFLUSSRATEN EINES GASES IN EINEM GASSFLUSS

DÉTERMINATION DU DEBIT VOLUMETRIQUE D'UN GAZ DANS UN FLUX GAZEUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**12.04.2017  Bulletin 2017/15**

(73) Proprietor: **Sensirion AG**
**8712 Stäfa (CH)**

(72) Inventors:
• **HORNUNG, Mark**
 **8712 Stäfa (CH)**

• **RÜEGG, Andreas**
 **8712 Stäfa (CH)**

(74) Representative: **Detken, Andreas**
 **Isler & Pedrazzini AG**
 **Giesshübelstrasse 45**
 **Postfach 1772**
 **8027 Zürich (CH)**

(56) References cited:
 **EP-A1- 2 574 918**    **EP-A1- 2 806 271**
 **EP-A2- 0 554 095**    **WO-A1-01/18500**
 **WO-A1-2015/075278**

EP 3 153 854 B1

**Description**

TECHNICAL FIELD

The present invention relates to a method, a gas sensor and a computer program for measuring a volumetric flow rate in a gas flow. The method can be employed in the context of determining a combustion parameter of a gas in a gas flow, for instance, a calorific value (heat of combustion per unit mass or per unit volume), a Wobbe index, or a methane number.

PRIOR ART

One traditional method of determining parameters that characterize the combustion behavior of complex gas mixtures such as natural gases includes an analysis of the gas composition by gas chromatography. This method is complex and expensive. Other methods are based on burning the gas and directly measuring the resulting heat of combustion, either in a qualitative manner with an open flame or in a quantitative manner by a calorimetric analysis with a specialized calorimeter. These methods are prone to errors and/or complicated.

Therefore, a number of methods have been proposed that aim at estimating combustion parameters of a gas without burning the gas, based on measurements of physical properties only.

For instance, in DE 101 22 039 A1 a gas is passed with a constant volumetric flow through a measurement tube. A heater, a first temperature sensor upstream of the heater, and a second temperature sensor downstream from the heater are provided on the measurement tube. A temperature difference between the temperature sensors is determined while the heater is operated at constant power. A calorific value of the gas is then calculated as a linear function of the temperature difference, using empirically determined coefficients. This method is simple, but rather inaccurate.

In DE 41 18 781 A1 a volumetric flow rate, a mass flow rate and a pressure difference across a laminar flow restrictor at isothermal conditions are determined. The mass flow rate is determined with a thermal flow sensor. Approximate values of the Wobbe index and of the heat of combustion are calculated from these quantities with empirically determined formulae. The method is relatively complicated, requiring active temperature control to keep the laminar flow restrictor at constant temperature.

In WO 2015/075278 a thermal flow sensor is employed to measure temperatures upstream and downstream from a heater. In addition, a pressure difference across a flow restrictor is measured. The heat conductivity and the microscopic force of the gas are determined from these measurements, and empirically determined correlation functions are employed to calculate a combustion parameter from these physical parameters. US 7,377,152 B2 also discloses that a thermal flow sensor is employed and that a pressure difference is measured across a flow restrictor to determine, inter alia, a combustion parameter. These methods require a pressure difference across a flow restrictor to be determined.

EP 2 086 271 A1 discloses a method for determining physical gas properties. The gas is passed through a critical nozzle and past a microthermal sensor. The pressure drop in a reservoir of gas flowing to the nozzle is measured. A first gas property factor is determined based on the measured pressure drop, and a second gas property factor is determined based on a flow signal generated by the microthermal sensor. A thermal conductivity of the gas is determined using the microthermal sensor. The first and/or second gas property factors and the thermal conductivity are correlated to determine the physical gas property.

EP 2 574 918 A1 discloses another method for determining physical gas properties. A microthermal sensor is used to determine a physical gas property that is complementary to a physical gas property measured by another sensor with respect to the ratio between flow velocity and heat diffusivity. In addition, the heat conductivity is determined using the microthermal sensor.

EP 0 554 095 discloses a method for determining thermophysical or thermochemical parameters of a fuel gas. The method utilizes a specified relationship between the characteristic specific heat and thermal conductivity at a reference condition of the fuel gas and the rate of change of these characteristics at the reference conditions.

US2013199290 discloses a related method of retrofitting a bellows gas meter with a measuring device comprising a thermal flow measuring sensor and using the signal of said sensor to calculate a flow velocity.

SUMMARY OF THE INVENTION

The present invention provides a method for measuring a volumetric flow rate in a gas flow according to claim 1. The method comprises:

passing the gas flow through at least one chamber that defines a variable volume, gas being alternatingly admitted into the chamber and being subsequently again expelled from the chamber, the alternate admission and expulsion causing oscillations of the gas flow;

detecting an oscillation signal by a thermal flow sensor, the oscillation signal being indicative of the oscillations of the gas flow; and

calculating the volumetric flow rate based on the oscillation signal.

In this method, the thermal flow sensor acts as the counter of admission and expulsion cycles of the volumetric flow sensor. If the volume change per cycle is known, the volumetric flow rate can be readily calculated from the thus-determined cycle rate.

Volumetric flow sensors having a chamber that defines a variable volume are often called bellows-type volumetric flow sensors. Such flow sensors are widely used as gas counters in residential and small commercial installations. They are usually equipped with a mechanical counter only. The presently proposed method is particularly useful if such a gas counter is to be retrofitted so as to enable electronic readout of the volumetric flow rate. Instead of providing a possibly complicated electronic counter on the existing mechanical counter mechanism, a simple and cheap thermal flow sensor can act as the cycle counter. The thermal flow sensor can then be additionally employed for other purposes, in particular, for determining combustion parameters. This method can thus readily be combined with a method of determining a combustion parameter, which method does not require sophisticated equipment, is simple to execute and accurate.

A corresponding method for determining a combustion parameter of the gas comprises:

operating the thermal flow sensor exposed to the gas flow;

calculating a heat transfer parameter and a heat capacity parameter of the gas from an output of the thermal flow sensor and from the volumetric flow rate; and

calculating the combustion parameter of the gas from the heat transfer parameter and the heat capacity parameter.

This method builds on two findings. The first finding is that combustion parameters (i.e., parameters that characterize the combustion behavior of the gas) of many gases, including most natural gases, can be very precisely predicted if a heat transfer parameter (i.e., a parameter that characterizes the heat transfer behavior of the gas, e.g., the thermal conductivity of the gas) and a heat capacity parameter (i.e., a parameter that characterizes the amount of energy required to heat a unit mass or a unit volume of the gas by a certain amount, e.g., its specific heat capacity per unit mass or unit volume) are known. The second finding is that both a heat transfer parameter and a heat capacity parameter can be readily determined by operating a thermal flow sensor exposed to the gas flow and by independently measuring a volumetric flow rate (or, equivalently, a flow velocity) of the gas flow. In particular, by operating the thermal flow sensor, it is possible to readily determine a heat transfer parameter as well as a mass flow parameter which depends, inter alia, on the mass flow rate and on the specific heat capacity per unit mass of the gas. By independently measuring a volumetric flow rate (or, equivalently, a flow velocity), a heat capacity parameter (in particular, the specific heat capacity per unit volume) can be extracted.

The method of the present invention is very simple and cost-efficient to implement. The method does not require sophisticated equipment. It can be employed in conjunction with any existing volumetric gas sensor, e.g., with any bellow-type gas counter as it is widely used in residential installations.

In preferred embodiments, the step of determining the heat transfer parameter and the heat capacity parameter comprises:

determining the heat transfer parameter and a mass flow parameter of the gas flow from the output of the thermal flow sensor;

calculating the heat capacity parameter from the mass flow parameter, from the flow velocity or volumetric flow rate, and from the heat transfer parameter.

In particular, the thermal flow sensor can comprise a heating element, a first temperature sensor upstream of the heating element, and a second temperature sensor downstream of the heating element. The method can then comprise:

determining a first temperature value at the first temperature sensor and a second temperature value at the second temperature sensor under action of the heating element; and

calculating the heat transfer parameter and the mass flow parameter taking into account said first and second temperature values.

In particular, the heat transfer parameter and the mass flow parameter can be calculated from the first and second temperature values or linear combinations thereof, more particularly, from their (possibly weighted) sum and their (possibly weighted) difference, using calibration data. In this case, it is preferred if the heating element is operated at a known and preferably constant heating power. However, there are other possible modes of operating a thermal flow sensor, and depending on the mode, other possibilities exist. For instance, the heating element can be actively controlled so as to keep one of the temperatures or their (possibly weighted) sum constant. In this case, the heat transfer parameter and the mass flow parameter can be derived from the heating power applied to the heating element (which will be strongly affected by the heat transfer behavior of the gas) and from the difference between the first and second temperature values (which will be strongly affected by the mass flow). Several other possibilities exist.

In particular, the heat transfer parameter that is determined in the method can be thermal conductivity, and the heat capacity parameter can be volumetric heat capacity (specific heat capacity per unit volume, or equivalently, the product of specific heat capacity per unit mass, multiplied by density).

In order to be able to calculate the combustion parameter at some defined standard conditions (i.e., at a reference temperature and reference pressure), the heat transfer parameter and the heat capacity parameter are preferably calculated for such predefined standard conditions, taking into account an actual gas temperature and an actual gas pressure, and the combustion parameter is then calculated from the heat transfer parameter and the heat capacity parameter at said standard conditions. To this end, the method of the present invention may include measuring the temperature and/or pressure of the gas. However, data relating to the temperature and/or pressure of the gas can also be obtained from other sources than a direct measurement. In particular, pressure may be known from a pressure regulator upstream from the volumetric flow sensor and the thermal flow sensor.

In order to calculate the heat capacity parameter at standard conditions, the step of calculating the heat capacity parameter can comprise:

calculating, from the measured volumetric flow rate, a standardized flow velocity or standardized volumetric flow rate at standard conditions, taking into account the actual gas temperature and the actual gas pressure; and employing the standardized flow velocity or standardized volumetric flow rate to obtain a value indicative of the heat capacity parameter at said standard conditions.

The combustion parameter can in particular be one of the following:

a calorific value, in particular, heat of combustion per unit volume;
Wobbe index; and
methane number.

In particular, the combustion parameter can be the thermodynamic heat of combustion, i.e., the energy released as heat when the gas undergoes complete combustion with oxygen under standard conditions, per unit volume. This combustion parameter is sometimes also referred to as the higher heating value per unit volume.

The combustion parameter is calculated from a correlation function having the heat transfer parameter and the heat capacity parameter as input variables. In particular, the correlation function can be a polynomial in these input variables. The coefficients of the polynomial can be determined empirically, as will be described by way of example in more detail below.

The present invention further provides a gas sensor for determining a volumetric flow rate of a gas in a gas flow, the gas sensor comprising:

a volumetric flow sensor of the bellows type comprising at least one chamber that defines a variable volume, the volumetric flow sensor being configured to alternatingly admit gas to the chamber and to subsequently again expel the gas from the chamber, the alternate admission and expulsion causing oscillations of the gas flow;
a thermal flow sensor configured to be exposed to the gas flow; and
a control device configured to carry out the following method:

detecting an oscillation signal based on signals from the thermal flow sensor, the oscillation signal being indicative of the oscillations of the gas flow; and
determining the volumetric flow rate based on the oscillation signal.

Furthermore, the gas sensor can be configured for determining a combustion parameter of the gas in the gas flow. To this end, the control device can be configured to carry out the following method:

operating the thermal flow sensor;
calculating a heat transfer parameter and a heat capacity parameter of the gas from an output of the thermal flow sensor and from the flow velocity or volumetric flow rate; and

calculating the combustion parameter of the gas from the heat transfer parameter and the heat capacity parameter.

Such a gas sensor can be made extremely compact. It is readily suitable to be employed in conjunction with any volumetric flow sensor that provides the indication of the flow velocity or the volumetric flow rate of the gas.

The gas sensor can comprise a volumetric flow sensor for determining the flow velocity or the volumetric flow rate of the gas in the gas flow. However, the gas sensor can instead also be configured to be retrofitted to an existing volumetric gas sensor, in particular, to an existing bellows-type gas counter.

The control device of the gas sensor can additionally be configured to carry out any of the method steps of the method of the present invention as described above.

As outlined above, the thermal flow sensor can comprise a heating element, a first temperature sensor upstream of the heating element, and a second temperature sensor downstream of the heating element. The control device can then be configured to carry out the following steps:

operating the thermal flow sensor to determine a first temperature value at the first temperature sensor and a second temperature value at the second temperature sensor under action of the heating element;

calculating the heat transfer parameter and a mass flow parameter of the gas taking into account said first and second temperature values or from their combinations; and

calculating the heat capacity parameter from the mass flow parameter, from the flow velocity or volumetric flow rate, and from the heat transfer parameter.

As outlined above, the combustion parameter is preferably calculated for some predefined standard conditions, taking into account the temperature and/or pressure of the gas. To this end, the gas sensor can comprise a temperature sensor and/or a pressure sensor. One or both of these sensors may be integrated with the thermal flow sensor.

The volumetric flow sensor of the present gas sensor can be a bellows-type volumetric flow sensor comprising at least one chamber that defines a variable volume, the volumetric flow sensor being configured to alternatingly admit gas to the chamber and to subsequently again expel the gas from the chamber, the alternate admission and expulsion causing oscillations of the gas flow. The control device can then be configured to carry out the following method:

detecting an oscillation signal based on signals from the thermal flow sensor, the oscillation signal being indicative of the oscillations of the gas flow; and

determining the volumetric flow rate based on the oscillation signal.

The invention also relates to a computer program comprising computer program code that, when carried out in a control device of a gas sensor according to the present invention, causes the control device to carry out the method of the present invention. The computer program can be provided in source code, in machine-executable code, or in any intermediate form of code like object code. It can be provided as a computer program product on a computer-readable medium in tangible form, e.g., on a CD-ROM or on a Flash ROM memory element, or it can be made available in the form of a network-accessible medium for download from one or more remote servers through a network.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

Fig. 1    shows a highly schematic functional diagram of a gas sensor according to an embodiment of the present invention;

Fig. 2    shows a schematic perspective view of a thermal flow sensor that can be employed in the present invention;

Fig. 3    shows a schematic cross-sectional view of a bellows-type volumetric flow sensor that can be employed in the present invention;

Fig. 4    shows a diagram illustrating the variations of the gas flow over time through a bellows-type volumetric flow sensor;

Fig. 5    shows a simplified block diagram of a digital control circuit that can be employed in the present invention;

Fig. 6    shows a flow diagram of a method according to an embodiment of the present invention;

Fig. 7    shows a diagram that correlates the calorific value of various gases as calculated from their thermal conductivity and heat capacity per unit volume at standard conditions to their calorific value as taken from literature.

DESCRIPTION OF PREFERRED EMBODIMENTS

General setup of gas sensor according to an embodiment

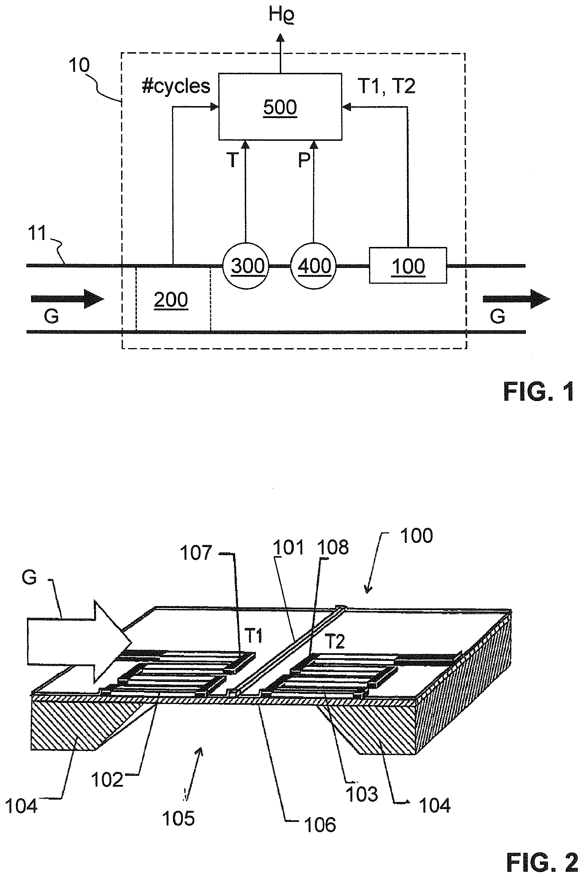

Figure 1 shows a highly schematic functional diagram of a gas sensor 10 according to an embodiment of the present invention. A gas flow G passes through a conduit 11. The gas sensor 10 comprises a volumetric flow sensor 200, a temperature sensor 300, a pressure sensor 400, and a thermal flow sensor 100. These sensors are connected to a control device 500. As will be explained in more detail below, the control device 500 receives the rate of displacement cycles of the volumetric flow sensor 200, an indicator of temperature T from the temperature sensor 300, an indicator of pressure P from the pressure sensor 400, and two temperature values upstream and downstream of a heating element from the thermal flow sensor 100. From these values, the control device 500 calculates, inter alia, an indicator of the calorific value of the gas in the gas flow G, in particular, its heat of combustion per unit volume $H_{\rho_0}$ at predefined standard conditions (e.g., 20 °C and 1013 mbar).

It is to be understood that Fig. 1 is only highly schematic. In particular, while it is advantageous for the thermal flow sensor to be arranged downstream from the volumetric gas sensor as shown, the order of the sensors in the conduit may generally be different than shown. In some embodiments, the temperature sensor and/or the pressure sensor may be integrated with the thermal flow sensor. The sensors 100, 200, 300 and 400 may be arranged in a common housing or may be arranged in separate housings. The thermal flow sensor 100 may be arranged directly in the main flow within the conduit 11, as shown, or it may be arranged in a bypass portion of the conduit 11. For instance, the conduit 11 and the thermal flow sensor 100 may be configured in the manner disclosed in WO 01/98736 A1, EP 2 527 779 A1 or WO 2012/021999 A1.

Thermal flow sensor

Figure 2 illustrates an example of a thermal flow sensor 100 that can be employed in conjunction with the present invention. The thermal flow sensor 100 comprises a resistive heater 101 arranged between a first temperature sensor 102 upstream of the heater 101 and a second temperature sensor 103 downstream from the heater 101. In the present embodiment, the temperature sensors 102, 103 are thermopiles; however, the invention can also be carried out with other types of temperature sensors, such as resistive temperature sensors. The thermal flow sensor 100 further comprises a substrate 104, preferably a semiconductor substrate, in particular a silicon substrate. The heater 101, the temperature sensors 102, 103, and optionally analog and digital circuitry (e.g. analog amplifiers, an A/D-converter, and a digital signal processor, not shown in Fig. 2) are integrated on a surface of the substrate 104. An opening or recess 105 in the substrate 104 has been manufactured e.g. by anisotropic etching; the recess 105 is spanned by a membrane 106. The temperature sensors 102, 103 as well as the heater 101 are arranged at least partially on the membrane 106 for good thermal insulation. At least the portion of the sensor 100 that comprises the heater 111 and the temperature sensors 112, 113 extends into the conduit 13, so as to be in thermal contact with the gas flow G. This type of thermal flow sensor is described in more detail in WO 01/98736 A1 and US 7,188,519 B2, and the disclosure of these documents is incorporated herein by reference in its entirety for teaching the setup of a thermal flow sensor.

The flow sensor 11 is operated as follows. The gas flow G is passed over first temperature sensor 102, heater 101 and second temperature sensor 103. Heater 101 is heated by an electric current. Thermal conductance through membrane 106 as well as through the gas in flow G leads to a temperature increase at inner contacts 107, 108 of the temperature sensors 102, 103, while the outer contacts remain at the bulk temperature of substrate 104. In the presence of a non-zero flow, the temperature distribution is asymmetric, and the temperature $T_1$ measured by first temperature sensor 102 will generally be lower than the temperature $T_2$ measured by second temperature sensor 103. A description how these values are processed further will be provided below.

Volumetric flow sensor

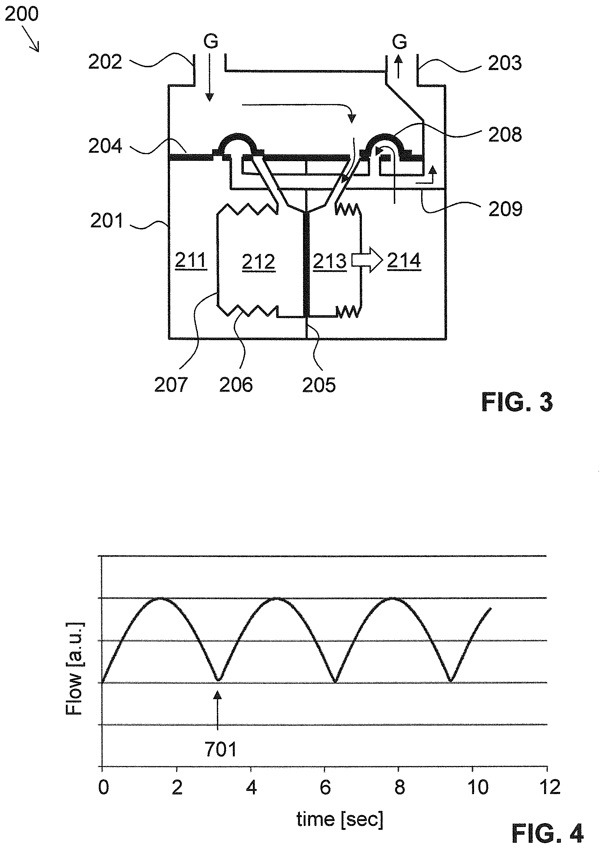

Figure 3 illustrates an example of a volumetric flow sensor 200 that can be employed in conjunction with the present invention. The volumetric flow sensor 200 of this embodiment is a positive-displacement sensor of the bellows type. Such sensors have been well known for a long time and are the most common form of residential gas meters. The sensor 200 comprises a housing 201 having an inlet 202 and an outlet 203 for the gas flow G. A horizontal wall 204 and a vertical wall 205 delimit two chambers 211, 214. In each of these chambers, an accordion-like bellows delimited by a zig-zag shaped foldable wall 206 and a rigid disk 207 is arranged. Each of the bellows defines a chamber 212, 213 having a variable volume. By expansion and contraction of chambers 212, 213 defined by the bellows, the available volume of the chambers 211, 214, respectively is also variable. The volumes of chambers 211, 212 are directly coupled: an increase of the volume of chamber 211 effects a corresponding decrease of the volume of chamber 212. Likewise, chambers 213, 214 are coupled. The horizontal wall 204 defines several orifices, two of which lead to an outlet pipe 209. Sliding valves 208 cover the orifices in such a manner that gas entering the flow sensor 200 is admitted into only one of chambers 211, 212, 213, 214 at a time, while gas from another chamber that is coupled to this chamber is expelled into the outlet pipe 209. By a pressure difference between the inlet 202 and the outlet 203, each of the bellows is alternatingly expanded and contracted. A mechanism (not illustrated in Fig. 3) ensures that the valves 208 open and close the orifices in such a manner that the measuring volume that is admitted into and expelled from each chamber per cycle of the expansion and contraction of the bellows is constant. By the mechanism, the oscillating movement of the bellows is transformed into a rotational movement and is mechanically transmitted to a counter. In one example not in accordance with the present invention, the counter directly transmits the cycle count to the control unit 500, and the control unit 500 calculates the volumetric flow rate from the cycle count. In another example not in accordance with the present invention, an electronic evaluation unit of volumetric flow sensor 200 directly outputs the volumetric flow rate. Since the entire gas flow has to pass through one of the chambers, the entire volume of gas passing through the sensor is measured.

In the above-described embodiments, the volumetric flow sensor 200 directly communicates with the control device 500. In accordance with the present invention, the expansion and contraction movement of the bellows is monitored indirectly by the thermal flow sensor 100 by detecting a typical oscillation pattern in the flow rate, as illustrated in Fig. 4. Flow through a bellows-type volumetric gas sensor is not constant over time, but is oscillatory, with a momentary minimum of the flow rate occurring each time one of the valves moves so as to admit gas to a different chamber (point 701 in Fig. 4). By detecting this typical flow pattern with the thermal flow sensor, the thermal flow sensor acts as a cycle counter for the volumetric flow sensor. In this case, no data connection between the volumetric flow sensor 200 and the control device 500 is needed. This is particularly advantageous in cases where the thermal flow sensor 100, the temperature sensor 300, the pressure sensor 400 and the control device 500 are retrofitted to an existing bellow-type volumetric flow sensor.

In yet other examples not in accordance with the present invention, the volumetric flow sensor can be an ultrasound flow sensor, as it is well known in the art. An ultrasound flow sensor can be used to directly determine a flow velocity of the gas flow.

Control device

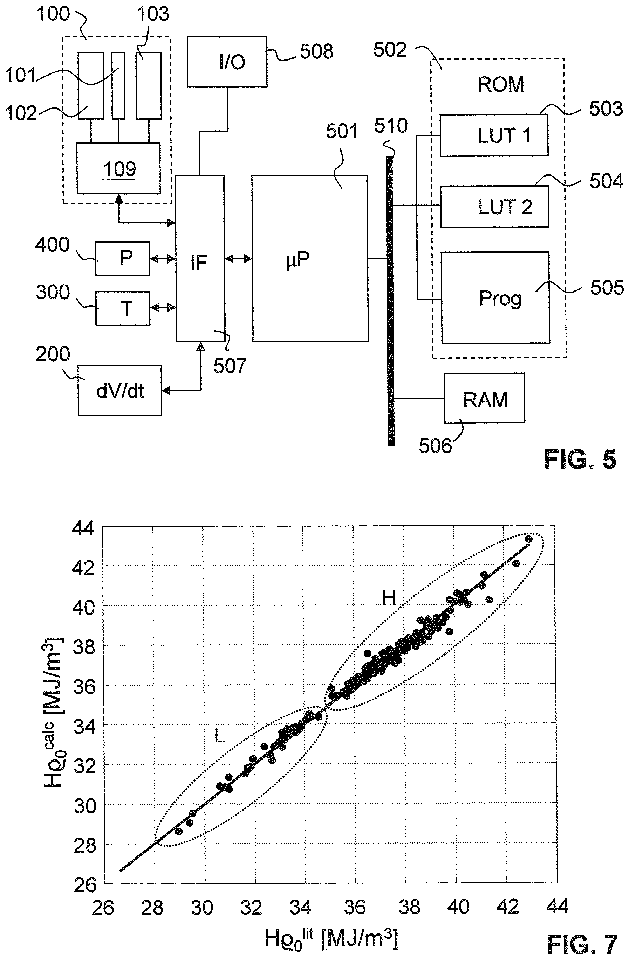

A simplified and highly schematic block diagram of a digital control device 500 is shown in Fig. 5. The control device comprises a processor (CPU, $\mu$P) 501, a non-volatile (e.g., Flash ROM) memory 502, and a volatile (RAM) memory 506. The processor 501 communicates with the memory modules 502, 506 via one more data buses 510. The non-volatile memory 502 stores, inter alia, plural sets of calibration data 503, 504 in the form of lookup tables LUT1, LUT2, as well as a machine-executable program 505 for execution in the processor 501. Lookup table LUT1 includes calibration data from calibration measurements with reference gases, as will be described in more detail below, while lookup table LUT2 includes coefficients of a polynomial expansion of a combustion parameter as a function of two physical parameters, as will also be described in more detail below.

Via a device interface (IF) 507, the processor 501 communicates with various peripherals, which may include, e.g., at least one input/output (I/O) interface 508 for interfacing with an external input/output device such as a keyboard and/or mouse, an LCD screen, etc., with the volumetric flow sensor 200, with the temperature sensor 300, with the pressure sensor 400, and with the thermal flow sensor 100 via its built-in digital circuitry 109.

Determination of combustion parameters

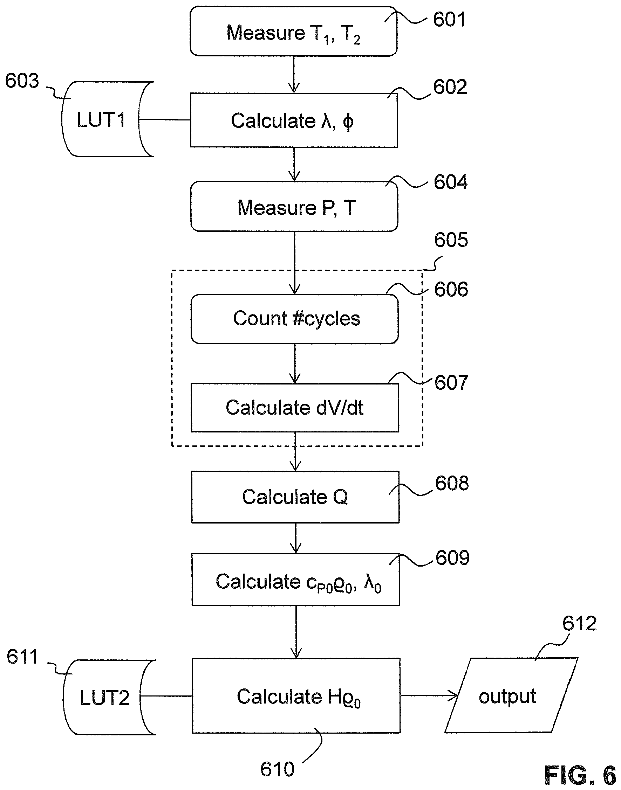

Combustion parameters such as calorific value (in particular, heat of combustion per unit volume at standard conditions $H\rho_0$), Wobbe index $I_W$ and methane number $N_M$ are determined as described in the following with reference to the flow diagram in Fig. 6.

In this embodiment, the calculation of the combustion parameters is based on a determination of thermal conductivity at standard conditions, $\lambda_0$, and volumetric heat capacity (i.e., heat capacity per unit volume) at standard conditions, $c_{P0}\rho_0$. The combustion parameters are calculated as a function of these variables, for instance, as a polynomial of these variables, as will be detailed below. It will therefore be described in the following how a thermal conductivity at standard conditions, $\lambda_0$, and volumetric heat capacity at standard conditions, $c_{P0}\rho_0$, are determined from measurements with the volumetric flow sensor and the thermal flow sensor, while taking temperature and pressure into account.

In step 601, the first and second temperatures $T_1$ and $T_2$ or linear combinations thereof are measured by the thermal flow sensor 100. In step 602, the thermal conductivity $\lambda$ and a mass flow parameter $\phi$ are calculated from these values, using lookup table LUT1 (box 603) as detailed in the following.

As linear combinations, the difference $\Theta_1 = T_2 - T_1$ and the sum $\Theta_2 = T_1 + T_2$ are employed, but also other linear combinations can be used. The first and second temperatures $T_1$ and $T_2$ and thereby also their linear combinations depend, inter alia, on the mass flow $\rho v$ and on the heat transfer properties of the gas, in particular, on its thermal

conductivity $\lambda$. For instance, the dependence of the difference and sum values $\Theta_1$ and $\Theta_2$ can be expressed as follows:

$$\Theta_i = F_i \left( \frac{c_P \rho v}{\lambda}, \lambda \right), i = 1,2 \qquad (1)$$

Here, $c_P$ is the specific heat capacity of the gas at constant pressure, $\rho$ is the density of the gas, and $v$ is the bulk velocity of the gas at the sensor location. This functional dependence is a consequence of the heat equation. The functions $F_i$ themselves depend on the details of the measurement setup, e.g., on the geometry of the conduit 11, on the geometry and position of the thermal flow sensor 100, etc. They may additionally depend on further properties of the gas, e.g., the Prandtl number of the gas flow, which in a first approximation can however be neglected. They may depend additionally explicitly on the temperature of the gas, e.g., through a temperature dependence of the Seebeck effect, of the resistive heater or of the heat conductivity of the sensor membrane. However, this temperature dependence is usually weak and can, in a first approximation, also be neglected.

Equation (1) defines a system of two equations with two unknown variables, namely, a mass flow parameter $\phi = c_P \rho v / \lambda$ and the thermal conductivity $\lambda$. This implies that it is generally possible to determine both the thermal conductivity $\lambda$ and the mass flow parameter $\phi$ from the linear combinations $\Theta_1$ and $\Theta_2$. In particular, the functional dependence of the thermal conductivity $\lambda$ on the temperature values $\Theta_1$ and $\Theta_2$ can be expressed as follows:

$$\lambda = f(\Theta_1, \Theta_2) \qquad (2)$$

In the present embodiment, the function f is determined through calibration measurements as follows. Two calibration gases $g_1$ and $g_2$ with known thermal conductivities $\lambda^{g1}$ and $\lambda^{g2}$, respectively, at a reference temperature are employed for the calibration measurements. Both gases $g_1$ and $g_2$ are sent, one at a time, through the gas sensor 10 while the mass flow rate $\rho v$ is varied throughout the relevant flow range, so as to determine a set of value triples ($\phi^{g1}$, $\Theta_1^{g1}$, $\Theta_2^{g1}$) for gas $g_1$ and ($\phi^{g2}$, $\Theta_1^{g2}$, $\Theta_2^{g2}$) for gas $g_2$. It should be noted that the mass flow parameter $\phi = c_P \rho v / \lambda$ depends both on the actual mass flow $\rho v$ (which is assumed to be known in the calibration measurements) as well as on gas-specific parameters $c_P$ and $\lambda$. These value triples are stored in lookup table LUT1. When a gas with unknown mass flow rate and unknown thermal conductivity is passed through the gas sensor 10, the values of the temperature difference $\Theta_1$ and of the sum $\Theta_2$ are determined for this gas flow, and the thermal conductivity $\lambda$ is calculated by means of linear interpolation of the $\Theta_2$ value at the given $\Theta_1$ value, using the calibration data stored in lookup table LUT1, as follows:

$$\lambda = x\lambda^{g1} + (1 - x)\lambda^{g2} \qquad (3)$$

with

$$x = \left[ \Theta_2 - \Theta_2^{g2}(\Theta_1) \right] / \left[ \Theta_2^{g1}(\Theta_1) - \Theta_2^{g2}(\Theta_1) \right]. \qquad (4)$$

Note that for this determination of the thermal conductivity $\lambda$ the actual flow rate or the value of the mass flow parameter $\phi$ does not need to be known. This procedure can be readily generalized to a case where more than two calibration gases are used for calibration, or to a case where only one calibration gas is used in conjunction with master data, as described in WO 2015/075278.

The difference value $\Theta_1$ or another linear combination is now used to quantify the mass flow. In particular, the mass flow parameter $\phi$, which corresponds to $c_P \rho v / \lambda$, is determined from Equation (1). To this end, Equation (1) is rewritten as follows:

$$\Theta_1 = F_1(\phi, \lambda) \qquad (5)$$

In practice, an estimate of the function $F_1$ for a given value of $\lambda$ can be determined with the aid of the above-described calibration measurements as follows. The dependence of the difference value $\Theta_1$ upon the flow parameter $\phi$

and the thermal conductivity $\lambda$ can be linearized in parameter $\lambda$ as follows:

$$\Theta_1(\phi) = F_1(\phi, \lambda) = u\Theta_1^{g1}(\phi) + (1-u)\Theta_1^{g2}(\phi) \qquad (6)$$

with

$$u = (\lambda - \lambda^{g2})/(\lambda^{g1} - \lambda^{g2}) \qquad (7)$$

$\Theta_1^{g1}(\phi)$ and $\Theta_1^{g2}(\phi)$ are known from the calibration measurements and can readily be interpolated for any value of $\phi$ from lookup table LUT1. Equation (6) thus defines a unique calibration curve $\Theta_1(\phi)$ for any given value of $\lambda$. Therefore, for any given measured value $\Theta_1$, Equation (6) implicitly defines the mass flow parameter $\phi = \phi(\Theta_1)$.

In order for the linearization in Equation (6) to represent a good approximation, the thermal conductivity $\lambda$ should preferably be sufficiently close to $\lambda^{g1}$ and $\lambda^{g2}$, which means that $\lambda$ should preferably be in the range above 50%, preferably above 80% of the smaller of $\lambda^{g1}$ and $\lambda^{g2}$ and below 150%, preferably below 125% of the larger of $\lambda^{g1}$ and $\lambda^{g2}$. More preferably $\lambda^{g1} \le \lambda \le \lambda^{g2}$, wherein $\lambda^{g1}$ and $\lambda^{g2}$ differ by not more than 20% of $\lambda^{g2}$.

Again, this procedure can be readily generalized to a case where more than two calibration gases are used for calibration, or to a case where only one calibration gas is used in conjunction with master data, as described in WO 2015/075278.

This procedure eventually leads to a determination of thermal conductivity $\lambda$ and of the mass flow parameter $\phi$, which corresponds to $c_P\rho v/\lambda$. The mass flow parameter depends not only on the flow velocity v, but also on the volumetric heat capacity $c_P\rho$ and on the thermal conductivity $\lambda$. Since the thermal conductivity $\lambda$ is now known, the above procedure enables a determination of the volumetric heat capacity $c_P\rho$ if the flow velocity v or a quantity that is proportional to the flow velocity, e.g., the volumetric flow, is known from an independent measurement, e.g., from a volumetric flow measurement. However, the resulting value $c_P\rho$ would depend on temperature and pressure because the gas density $\rho$ strongly depends on these quantities, and because the specific heat $c_P$ is temperature-dependent for real gases. In order to determine combustion parameters, it is however desirable to use values at some predefined standard conditions. It is therefore desirable to transform the volumetric heat capacity that has been determined in this manner into a volumetric heat capacity at standard conditions, i.e., at a reference temperature $T_0$ and a reference pressure $P_0$. This can be done in various ways. One possibility will be described in the following with reference to steps 604-609 in Fig. 6.

In step 604, the actual gas pressure $P$ and the actual gas temperature T in the conduit 11 are determined. In step 605, the volumetric flow rate $dV/dt$ is measured, e.g., by a positive displacement flow sensor. For instance, if a bellows-type volumetric flow sensor is used, the number of cycles of the bellows may be determined (step 606) by the volumetric flow sensor itself or by the thermal flow sensor, and the volumetric flow rate $dV/dt$ may be calculated from this (step 607). Alternatively, it is possible to measure the flow velocity, e.g., by an ultrasound flow sensor.

In step 608, the volumetric flow $dV/dt$ is transformed into a standardized volumetric flow $Q$ at a reference temperature $T_0$ and reference pressure $P_0$ as follows:

$$Q = \left(\frac{P}{P_0}\right)\left(\frac{T_0}{T}\right) dV/dt \qquad (8)$$

A similar transformation can also be carried out for the flow velocity if the flow velocity has been measured. Strictly speaking, Equation (8) is only valid in the approximation of an ideal gas. For real gases, a more complicated dependency may be used instead of Equation (8). However, Equation (8) will generally be sufficiently precise if the actual gas temperature $T$ and actual gas pressure $P$ do not too strongly deviate from the reference values $T_0$ and $P_0$.

Now the mass flow parameter $\phi$ in Equation (5) can be rewritten as follows, using the parameter $Q$:

$$\phi = \frac{c_P(T)\rho(P,T)v}{\lambda(T)} \sim \frac{c_P(T)\rho(P_0,T_0)Q}{\lambda(T)} \qquad (9)$$

The factor of proportionality generally depends on the Reynolds number. Note that the density is expressed on the right hand side of Equation (9) as a density at standard conditions, $\rho(P_0,T_0)$, while the specific heat $c_P(T)$ and the thermal conductivity $\lambda(T)$ are still expressed at the actual measurement temperature.

Subsequently, a quantity $c_0 = \phi\lambda(T)/Q$ is calculated. The quantity $c_0$ is a volumetric heat capacity parameter that is proportional to the value of $c_P(T)\rho(P_0,T_0)$:

$$c_0 = \phi \cdot \lambda(T)/Q \sim c_P(T)\rho(P_0,T_0) \tag{10}$$

The right hand side of Equation (10) is independent of the gas pressure $P$, but still depends on temperature $T$ through $c_P(T)$. A temperature compensation of $c_0$ can however be achieved rather easily, since the temperature coefficients of the specific heat $c_P(T)$ are very similar for most combustible gases in a particular class, e.g., for all natural gases. In this manner, the volumetric specific heat at standard conditions, $c_{P0}\rho_0 = c_P(T_0)\rho(P_0, T_0)$, is obtained. Likewise, a temperature compensation of the thermal conductivity $\lambda$ can be carried out rather easily, since also the temperature coefficients of the thermal conductivity are very similar for most combustible gases in a particular class. In this manner, the thermal conductivity at standard temperature, $\lambda_0 = \lambda(T_0)$, is obtained. This completes step 609 in Fig. 6.

In step 610, combustion parameters such as calorific value (in particular, heat of combustion per unit volume at standard conditions $H\rho_0$, Wobbe index $I_W$ and methane number $N_M$) are determined as follows. The combustion parameters are calculated as a function of the variables $c_{P0}\rho_0$ and $\lambda_0$, for instance, as a polynomial of these variables:

$$H\rho_0 = A_1 + B_1\lambda_0 + C_1\lambda_0^2 + D_1(c_{P0}\rho_0) + E_1(c_{P0}\rho_0)^2 + F_1\lambda(c_{P0}\rho_0) \tag{11}$$

$$I_W = A_2 + B_2\lambda_0 + C_2\lambda_0^2 + D_2(c_{P0}\rho_0) + E_2(c_{P0}\rho_0)^2 + F_2\lambda(c_{P0}\rho_0) \tag{12}$$

$$N_M = A_3 + B_3\lambda_0 + C_3\lambda_0^2 + D_3(c_{P0}\rho_0) + E_3(c_{P0}\rho_0)^2 + F_3\lambda(c_{P0}\rho_0) \tag{13}$$

The coefficients $A_i$, $B_i$, $C_i$, $D_i$, $E_i$, $F_i$, $i = 1,2,3$, can be determined by a regression analysis on a data set of known gases. For instance, the "Report on gas composition range in Europe" (available under http://www.ingas-eu.org/docs/DB0.1.pdf, downloaded on October 1, 2015) contains the combustion parameters $H\rho_0$, $I_W$ and $N_M$ for a large number of natural gases, together with their compositions. If the composition is known, its gas-specific physical parameters $\lambda_0$ and $c_{P0}\rho_0$ can be calculated by means of commercially available programs such as Refprop NIST (available under http://www.nist.gov/srd/nist23.cfm, visited on October 1, 2015) or PPDS (available under http://www.tuv-sud.co.uk/uk-en/about-tuev-sued/tuev-sued-in-the-uk/nel/ppds-thermodynamic-properties-suite, visited on October 1, 2015). Thereby, a regression analysis for Equations (11), (12) and (13) can be carried out for a large number of gases.

Such a regression analysis for the calorific value $H\rho_0$ was carried out for 650 natural gases and led to the following values for coefficients $A_1$, $B_1$, $C_1$, $D_1$, $E_1$, $F_1$:

$A_1$ = -1349 MJ/m$^3$
$B_1$ = 1471 MJ/m$^3$
$C_1$ = -409 MJ/m$^3$
$D_1$ = 1189 MJ/m$^3$
$E_1$ = -245 MJ/m$^3$
$F_1$ = -620 MJ/m$^3$.

Here, the physical parameters $\lambda_0$ and $c_{P0}\rho_0$ were expressed as dimensionless variables by dividing by the respective physical parameter of methane; therefore, all coefficients have the dimension of energy per unit volume.

The coefficients $A_i$, $B_i$, $C_i$, $D_i$, $E_i$, $F_i$, $i = 1,2,3$, are stored in lookup table LUT2 (box 611).

In step 612, the resulting combustion parameters are outputted.

Figure 7 illustrates the quality of a regression analysis for 650 natural gases, including both so-called L gases and H gases. On the horizontal axis, the "true" calorific value per unit volume at standard conditions, $H\rho_0^{lit}$, as taken from available reports, is shown; on the vertical axis, calculated values $H\rho_0^{calc}$ obtained by Equation (11) are shown. The correlation between these values is remarkable, considering that a chemical property (heat of combustion) is calculated purely from physical properties (thermal conductivity and volumetric heat capacity).

In alternative embodiments, the thermal conductivity $\lambda = \lambda(T)$ and the specific heat per unit volume, $c_P\rho = c_P(T)\rho(P,T)$, can be determined at the actual temperature $T$ and pressure $P$, by using the above procedure, employing the actual volumetric flow rate $dV/dt$ rather than the standardized volumetric flow rate $Q$ and skipping temperature compensation of $c_P$ and $A$. Also in this case equations of the same form as equations (11)-(13) can be used; however, the coefficients $A_i$, $B_i$, $C_i$, $D_i$, $E_i$, $F_i$, $i = 1,2,3$, would have to be known for the actual pressure and temperature. If pressure and temperature are not constant, lookup table LUT2 would correspondingly contain the coefficients at more than one temperature and pressure and would thus be more complex.

All or part of control device 500 may be implemented as a dedicated digital signal processor (DSP) or as a general-purpose computer with a processor that executes software causing the processor to carry out the above-described steps. Consequently, the presently described method can be embodied in a computer program for execution on a general-purpose computer or on a dedicated DSP.

While there are shown and described presently preferred embodiments of the invention, it is to be understood that the invention is not limited thereto but may be otherwise variously embodied and practiced within the scope of the following claims.

**Claims**

1. A method for measuring a volumetric flow rate ($dV/dt$) of a gas in a gas flow (G), the method comprising:
   passing the gas flow (G) through at least one chamber (211, 212, 213, 214) that defines a variable volume, gas being alternatingly admitted into the chamber (211, 212, 213, 214) and being subsequently expelled from the chamber (211, 212, 213, 214), the alternate admission and expulsion causing oscillations of the gas flow (G); **characterised by** that the method comprises the steps of:

   detecting an oscillation signal with a thermal flow sensor (100), the oscillation signal being indicative of the oscillations of the gas flow (G); and
   calculating the volumetric flow rate ($dV/dt$) based on the oscillation signal.

2. The method of claim 1, the method comprising:

   calculating a heat transfer parameter ($\lambda_0$) and a heat capacity parameter ($c_{p0}\rho_0$) of the gas from an output of the thermal flow sensor (100) and from the volumetric flow rate ($dV/dt$); and
   calculating a combustion parameter ($H\rho$; $I_W$; $N_M$) of the gas from the heat transfer parameter ($\lambda_0$) and the heat capacity parameter ($c_{p0}\rho_0$).

3. The method of claim 2, wherein determining the heat transfer parameter ($\lambda_0$) and the heat capacity parameter ($c_{p0}\rho_0$) comprises:

   determining the heat transfer parameter ($\lambda_0$) and a mass flow parameter ($\phi$) of the gas flow from the output of the thermal flow sensor;
   calculating the heat capacity parameter ($c_{p0}\rho_0$) from the mass flow parameter ($\phi$), from the volumetric flow rate ($dV/dt$), and from the heat transfer parameter ($\lambda_0$).

4. The method of claim 3, wherein the thermal flow sensor (100) comprises a heating element (101), a first temperature sensor (102) upstream of the heating element (101), and a second temperature sensor (103) downstream of the heating element (101), and wherein the method comprises:

   determining a first temperature value ($T_1$) at the first temperature sensor (102) and a second temperature value ($T_2$) at the second temperature sensor (103) under action of the heating element (101);
   calculating the heat transfer parameter ($\lambda_0$) and the mass flow parameter ($\phi$) taking into account said first and second temperature values ($T_1$, $T_2$).

5. The method of any one of claims 2-4,

   wherein the heat transfer parameter ($\lambda_0$) is thermal conductivity, and
   wherein the heat capacity parameter ($c_{p0}\rho_0$) is a volumetric heat capacity of the gas.

6. The method of any one of claims 2-5, wherein the heat transfer parameter ($\lambda_0$) and the heat capacity parameter ($c_{p0}\rho_0$) are calculated for predefined standard conditions, taking into account an actual gas temperature ($T$) and an actual gas pressure ($P$), and wherein the combustion parameter ($H\rho$; $I_W$; $N_M$) of the gas is calculated from the heat

transfer parameter ($\lambda_0$) and the heat capacity parameter ($c_{p0}\rho_0$) at said standard conditions.

7. The method of claim 6, wherein calculating the heat capacity parameter ($c_{p0}\rho_0$) comprises:

calculating, from the measured volumetric flow rate ($dV/dt$), a standardized flow velocity or standardized volumetric flow rate ($Q$) at standard conditions, taking into account the actual gas temperature ($T$) and the actual gas pressure ($P$); and
employing the standardized flow velocity or standardized volumetric flow rate ($Q$) to obtain a value indicative of the heat capacity parameter ($c_{p0}\rho_0$) at said standard conditions.

8. The method of any one of claims 2-7, wherein the combustion parameter ($H\rho$; $I_W$; $N_M$) is one of the following:

a calorific value, in particular, heat of combustion per unit volume ($H\rho$);
Wobbe index ($I_W$); and
methane number ($N_M$).

9. The method of any one of claims 2-8, wherein the combustion parameter ($H\rho$; $I_W$; $N_M$) is calculated as a polynomial of the heat transfer parameter ($\lambda_0$) and the heat capacity parameter ($c_{p0}\rho_0$), the polynomial having empirically determined coefficients.

10. A gas sensor for determining a volumetric flow rate ($dV/dt$) of a gas in a gas flow (G), the gas sensor comprising:

a volumetric flow sensor (200) of the bellows type comprising at least one chamber (211, 212, 213, 214) that defines a variable volume, the volumetric flow sensor (200) being configured to alternatingly admit gas to the chamber (211, 212, 213, 214) and to subsequently again expel the gas from the chamber (211, 212, 213, 214), the alternate admission and expulsion causing oscillations of the gas flow (G);
a thermal flow sensor (100) configured to be exposed to the gas flow (G); and **characterised by**
a control device (500) configured to carry out the following method:

detecting an oscillation signal based on signals from the thermal flow sensor (100), the oscillation signal being indicative of the oscillations of the gas flow (G); and
determining the volumetric flow rate ($dV/dt$) based on the oscillation signal.

11. The gas sensor of claim 10, wherein the control device (500) is configured to carry out the following method for determining a combustion parameter ($H\rho$; $I_W$; $N_M$) of the gas:

calculating a heat transfer parameter ($\lambda_0$) and a heat capacity parameter ($c_{p0}\rho_0$) of the gas from an output of the thermal flow sensor (100) and from the volumetric flow rate ($dV/dt$); and
calculating the combustion parameter ($H\rho$; $I_W$; $N_M$) of the gas from the heat transfer parameter ($\lambda_0$) and the heat capacity parameter ($c_{p0}\rho_0$).

12. The gas sensor of claim 11,
wherein the thermal flow sensor (100) comprises a heating element (101), a first temperature sensor (102) upstream of the heating element (101), and a second temperature sensor (103) downstream of the heating element (101), and wherein the control device (500) is configured to carry out the following steps:

operating the thermal flow sensor (100) to determine a first temperature value ($T_1$) at the first temperature sensor (102) and a second temperature value ($T_2$) at the second temperature sensor (103) under action of the heating element (101);
calculating the heat transfer parameter ($\lambda_0$) and a mass flow parameter ($\phi$) of the gas, taking into account said first and second temperature values ($T_1$, $T_2$); and
calculating the heat capacity parameter ($c_{p0}\rho_0$) from the mass flow parameter ($\phi$), from the volumetric flow rate ($dV/dt$), and from the heat transfer parameter ($\lambda_0$).

13. A method of retrofitting an existing bellows-type gas counter (200), wherein the gas counter (200) comprises at least one chamber (211, 212, 213, 214) that defines a variable volume, the gas counter (200) being configured to alternatingly admit gas to the chamber (211, 212, 213, 214) and to subsequently again expel the gas from the chamber (211, 212, 213, 214), the alternate admission and expulsion causing oscillations of the gas flow (G),

the method comprising retrofitting the gas counter (200) with a thermal flow sensor (100) configured to be exposed to the gas flow (G) and with a control device (500) that is configured to carry out the following method:

detecting an oscillation signal based on signals from the thermal flow sensor (100), the oscillation signal being indicative of the oscillations of the gas flow (G); and
determining the volumetric flow rate ($dV/dt$) based on the oscillation signal.

**14.** A computer program comprising computer program code that, when carried out in a control device (500) of a gas sensor according to any one of claims 10-12, causes the control device (500) to carry out a method according to any one of claims 1-9.

**Patentansprüche**

**1.** Verfahren zur Messung eines Volumenstroms ($dV/dt$) eines Gases in einem Gasstrom (G), wobei das Verfahren umfasst:

Leiten des Gasstroms (G) durch mindestens eine Kammer (211, 212, 213, 214), die ein variables Volumen definiert, wobei abwechselnd Gas in die Kammer (211, 212, 213, 214) eingelassen und anschliessend aus der Kammer (211, 212, 213, 214) ausgestossen wird, wobei das abwechselnde Einlassen und Ausstossen Oszillationen des Gasstroms (G) bewirkt;
**dadurch gekennzeichnet, dass** das Verfahren die Schritte umfasst von:

Erfassen eines Oszillationssignals mit einem thermischen Durchflusssensor (100), wobei das Oszillationssignal indikativ für die Oszillationen des Gasstroms (G) ist; und
Berechnen des Volumenstroms ($dV/dt$) basierend auf dem Oszillationssignal.

**2.** Verfahren nach Anspruch 1, wobei das Verfahren umfasst:

Berechnen eines Wärmeübertragungsparameters ($\lambda_0$) und eines Wärmekapazitätsparameters ($c_{p0}\rho_0$) des Gases aus einer Ausgabe des thermischen Durchflusssensors (100) und aus dem Volumenstrom ($dV/dt$); und
Berechnen eines Verbrennungsparameters ($H\rho$; $I_W$; $N_M$) des Gases aus dem Wärmeübertragungsparameter ($\lambda_0$) und dem Wärmekapazitätsparameter ($c_{p0}\rho_0$).

**3.** Verfahren nach Anspruch 2, wobei die Bestimmung des Wärmeübertragungsparameters ($\lambda_0$) und des Wärmekapazitätsparameters ($c_{p0}\rho_0$) umfasst:

Bestimmen des Wärmeübertragungsparameters ($\lambda_0$) und eines Massendurchflussparameters ($\phi$) des Gasstroms aus der Ausgabe des thermischen Durchflusssensors;
Berechnen des Wärmekapazitätsparameters ($c_{p0}\rho_0$) aus dem Massendurchflussparameter ($\phi$), aus dem Volumenstrom ($dV/dt$) und aus dem Wärmeübertragungsparameter ($\lambda_0$).

**4.** Verfahren nach Anspruch 3, wobei der thermische Durchflusssensor (100) ein Heizelement (101), einen ersten Temperatursensor (102) stromaufwärts des Heizelements (101) und einen zweiten Temperatursensor (103) stromabwärts des Heizelements (101) umfasst, und wobei das Verfahren umfasst:

Ermitteln eines ersten Temperaturwerts ($T_1$) an dem ersten Temperatursensor (102) und eines zweiten Temperaturwertes ($T_2$) am zweiten Temperatursensor (103) unter Einwirkung des Heizelements (101);
Berechnen des Wärmeübertragungsparameters ($\lambda_0$) und des Massendurchflussparameters ($\phi$) unter Berücksichtigung der ersten und zweiten Temperaturwerte ($T_1$, $T_2$).

**5.** Verfahren nach einem der Ansprüche 2-4,

wobei der Wärmeübertragungsparameter ($\lambda_0$) die Wärmeleitfähigkeit ist, und
wobei der Wärmekapazitätsparameter ($c_{p0}\rho_0$) eine volumetrische Wärmekapazität des Gases ist.

**6.** Verfahren nach einem der Ansprüche 2-5, wobei der Wärmeübertragungsparameter ($\lambda_0$) und der Wärmekapazitätsparameter ($c_{p0}\rho_0$) für vordefinierte Standardbedingungen berechnet werden, wobei eine tatsächliche Gastemperatur ($T$) und ein tatsächlicher Gasdruck ($P$) berücksichtigt werden, und wobei der Verbrennungsparameter ($H\rho$; $I_W$; $N_M$) des Gases aus dem Wärmeübertragungsparameter ($\lambda_0$) und dem Wärmekapazitätsparameter ($c_{p0}\rho_0$) bei den genannten Standardbedingungen berechnet wird.

7. Verfahren nach Anspruch 6, wobei das Berechnen des Wärmekapazitätsparameters ($c_{p0}\rho_0$) umfasst:

> Berechnen, aus dem gemessenen Volumenstrom ($dV/dt$), einer standardisierten Strömungsgeschwindigkeit oder eines standardisierten Volumenstroms ($Q$) bei Standardbedingungen, unter Berücksichtigung der tatsächlichen Gastemperatur ($T$) und des tatsächlichen Gasdrucks ($P$); und
> Verwenden der standardisierten Strömungsgeschwindigkeit oder des standardisierten Volumenstroms ($Q$), um einen Wert zu erhalten, der indikativ für den Wärmekapazitätsparameter ($c_{p0}\rho_0$) bei den genannten Standardbedingungen ist.

8. Verfahren nach einem der Ansprüche 2-7, wobei der Verbrennungsparameter ($H\rho$; $I_W$; $N_M$) einer der folgenden ist:

> ein Brennwert, insbesondere die Verbrennungswärme pro Volumeneinheit ($H\rho$);
> Wobbe-Index ($I_W$); und
> Methanzahl ($N_M$).

9. Verfahren nach einem der Ansprüche 2-8, wobei der Verbrennungsparameter ($H\rho$; $I_W$; $N_M$) als ein Polynom aus dem Wärmeübertragungsparameter ($\lambda_0$) und des Wärmekapazitätsparameters ($c_{p0}\rho_0$) berechnet wird, wobei das Polynom empirisch bestimmte Koeffizienten aufweist.

10. Gassensor zum Bestimmen eines Volumenstroms ($dV/dt$) eines Gases in einem Gasstrom (G), wobei der Gassensor umfasst:

> einen Volumenstromsensor (200) des Balgentyps, der mindestens eine Kammer (211, 212, 213, 214) umfasst, die ein variables Volumen definiert, wobei der Volumenstromsensor (200) derart ausgebildet ist, dass er abwechselnd Gas in die Kammer (211, 212, 213, 214) einlässt und das Gas anschliessend wieder aus der Kammer (211, 212, 213, 214) ausstösst, wobei das abwechselnde Einlassen und Ausstossen Oszillationen des Gasstromes (G) verursacht;
> einen thermischen Durchflusssensor (100), der ausgebildet ist, um dem Gasstrom (G) ausgesetzt zu sein; und
> **gekennzeichnet durch**
> eine Steuervorrichtung (500), die derart konfiguriert ist, dass sie das folgende Verfahren durchführt:
>
>> Erfassen eines Oszillationssignals basierend auf Signalen vom thermischen Durchflusssensor (100), wobei das Oszillationssignal indikativ für die Oszillationen des Gasstroms (G) ist; und
>> Bestimmen des Volumenstroms ($dV/dt$) basierend auf dem Oszillationssignal.

11. Gassensor nach Anspruch 10, wobei die Steuervorrichtung (500) konfiguriert ist, um das folgende Verfahren zur Bestimmung eines Verbrennungsparameters ($H\rho$; $I_W$; $N_M$) des Gases durchzuführen:

> Berechnen eines Wärmeübertragungsparameters ($\lambda_0$) und eines Wärmekapazitätsparameters ($c_{p0}\rho_0$) des Gases aus einer Ausgabe des thermischen Durchflusssensors (100) und aus dem Volumenstrom ($dV/dt$); und
> Berechnen des Verbrennungsparameters ($H\rho$; $I_W$; $N_M$) des Gases aus dem Wärmeübertragungsparameter ($\lambda_0$) und dem Wärmekapazitätsparameter ($c_{p0}\rho_0$).

12. Gassensor nach Anspruch 11,
wobei der thermische Durchflusssensor (100) ein Heizelement (101), einen ersten Temperatursensor (102) stromaufwärts des Heizelements (101) und einen zweiten Temperatursensor (103) stromabwärts des Heizelements (101) umfasst, und wobei die Steuervorrichtung (500) so konfiguriert ist, dass sie die folgenden Schritte ausführt:

> Betreiben des thermischen Durchflusssensors (100) zur Bestimmung eines ersten Temperaturwertes ($T_1$) an dem ersten Temperatursensor (102) und eines zweiten Temperaturwertes ($T_2$) am zweiten Temperatursensor (103) unter Einwirkung des Heizelements (101);
> Berechnen des Wärmeübertragungsparameters ($\lambda_0$) und eines Massendurchflussparameters ($\phi$) des Gases unter Berücksichtigung der ersten und zweiten Temperaturwerte ($T_1$, $T_2$); und
> Berechnen des Wärmekapazitätsparameters ($c_{p0}\rho_0$) aus dem Massendurchflussparameter ($\phi$), aus dem Volu-

menstrom (*dV/dt*) und aus dem Wärmeübertragungsparameter ($\lambda_0$).

13. Verfahren zum Nachrüsten eines vorhandenen Balgengaszählers (200), wobei der Gaszähler (200) mindestens eine Kammer (211, 212, 213, 214) aufweist, die ein variables Volumen definiert, wobei der Gaszähler (200) derart ausgebildet ist, dass er abwechselnd Gas in die Kammer (211, 212, 213, 214) einlässt und das Gas anschliessend wieder aus der Kammer (211, 212, 213, 214) ausstösst, wobei das abwechselnde Einlassen und Ausstossen Oszillationen des Gasstroms (G) verursacht,
wobei das Verfahren das Nachrüsten des Gaszählers (200) mit einem thermischen Durchflusssensor (100), der ausgebildet ist, dem Gasstrom (G) ausgesetzt zu werden, und mit einer Steuervorrichtung (500), umfasst, die derart konfiguriert ist, dass sie das folgende Verfahren ausführt:

Erfassen eines Oszillationssignals basierend auf Signalen vom thermischen Durchflusssensor (100), wobei das Oszillationssignal indikativ für die Oszillationen des Gasstromes (G) ist; und
Bestimmen des Volumenstroms (*dV/dt*) basierend auf dem Oszillationssignals.

14. Computerprogramm umfassend Computerprogrammcode, der, wenn er in einer Steuervorrichtung (500) eines Gassensors nach einem der Ansprüche 10-12 ausgeführt wird, die Steuervorrichtung (500) veranlasst, ein Verfahren nach einem der Ansprüche 1-9 auszuführen.

## Revendications

1. Un procédé pour déterminer un débit volumétrique (*dV/dt*) d'un gaz dans un flux de gaz (G), le procédé comprenant: faire passer le flux de gaz (G) à travers au moins une chambre (211, 212, 213, 214) qui définit un volume variable, le gaz étant alternativement admis dans la chambre (211, 212, 213, 214) et étant ensuite expulsé de la chambre (211, 212, 213, 214), l'admission et l'expulsion alternées provoquant des oscillations du flux de gaz (G); **caractérisé en ce que** le procédé comprend les étapes de:

détecter un signal d'oscillation avec un capteur de flux thermique (100), le signal d'oscillation étant indicatif des oscillations du flux de gaz (G); et
calculer le débit volumétrique (*dV/dt*) sur la base du signal d'oscillation.

2. Le procédé selon la revendication 1, le procédé comprenant:

calculer un paramètre de transfert de chaleur ($\lambda_0$) et d'un paramètre de capacité thermique ($c_p\rho_0$) du gaz provenant d'une sortie du capteur de débit thermique (100) et du débit volumétrique (*dV/dt*); et
calculer un paramètre de combustion ($H_\rho$; $I_W$; $N_M$) du gaz à partir du paramètre de transfert de chaleur ($\lambda_0$) et du paramètre de capacité thermique ($c_p\rho_0$).

3. Le procédé selon la revendication 2, dans lequel la détermination du paramètre de transfert de chaleur ($\lambda_0$) et du paramètre de capacité thermique ($c_p\rho_0$) comprend:

déterminer le paramètre de transfert de chaleur ($\lambda_0$) et un paramètre de débit massique ($\phi$) du flux de gaz à partir de la sortie du capteur de flux thermique;
calculer le paramètre de capacité thermique ($c_p\rho_0$) à partir du paramètre de débit massique ($\phi$), du débit volumétrique (*dV/dt*) et du paramètre de transfert de chaleur ($\lambda_0$).

4. Le procédé selon la revendication 3, dans lequel le capteur de flux thermique (100) comprend un élément chauffant (101), un premier capteur de température (102) en amont de l'élément chauffant (101), et un second capteur de température (103) en aval de l'élément chauffant (101), et dans lequel le procédé comprend:

déterminer une première valeur de température ($T_1$) au niveau du premier capteur de température (102) et une seconde valeur de température ($T_2$) au niveau du second capteur de température (103) sous l'action de l'élément chauffant (101);
calculer le paramètre de transfert de chaleur ($\lambda_0$) et le paramètre de débit massique ($\phi$) en tenant compte des dites première et seconde valeurs de température ($T_1$, $T_2$).

5. Le procédé selon l'une quelconque des revendications 2 à 4, dans lequel le paramètre de transfert de chaleur ($\lambda_0$)

est la conductivité thermique, et dans lequel le paramètre de capacité thermique ($c_p\rho_0$) est une capacité calorifique volumétrique du gaz.

6. Le procédé selon l'une quelconque des revendications 2 à 5, dans lequel le paramètre de transfert de chaleur ($\lambda_0$) et le paramètre de capacité thermique ($c_p\rho_0$) sont calculés pour des conditions standard prédéfinies, en tenant compte d'une température de gaz réelle (T) et d'une pression de gaz réelle (P), et dans lequel le paramètre de combustion ($H\rho$; $I_W$; $N_M$) du gaz est calculé à partir du paramètre de transfert de chaleur ($\lambda_0$) et du paramètre de capacité thermique ($c_p\rho_0$) auxdites conditions standard.

7. Le procédé selon la revendication 6, dans lequel le calcul du paramètre de capacité thermique ($c_p\rho_0$) comprend:

   calculer, à partir du débit volumétrique ($dV/dt$) mesuré, une vitesse d'écoulement standardisée ou un débit volumétrique standardisé (Q) aux conditions standard, en tenant compte de la température réelle du gaz (T) et du gaz réel pression (P); et

   employant la vitesse d'écoulement standardisée ou le débit volumétrique standardisé (Q) pour obtenir une valeur indicative du paramètre de capacité thermique ($c_p\rho_0$) auxdites conditions standard.

8. Le procédé selon l'une quelconque des revendications 2 à 7, dans lequel le paramètre de combustion ($H_\rho$; $I_W$; $N_M$) est l'un des suivants:

   un valeur calorifique, en particulier, la chaleur de combustion par unité de volume ($H\rho$);
   un indice de Wobbe ($I_W$); et
   un indice de méthane ($N_M$).

9. Le procédé selon l'une quelconque des revendications 2 à 8, dans lequel le paramètre de combustion ($H\rho$; $I_W$; $N_M$) est calculé comme un polynôme du paramètre de transfert de chaleur ($\lambda_0$) et du paramètre de capacité thermique ($c_p\rho_0$), le polynôme ayant des coefficients déterminés empiriquement.

10. Un capteur de gaz pour déterminer un débit volumétrique ($dV/dt$) d'un gaz dans un écoulement de gaz (G), le capteur de gaz comprenant:

   un capteur de débit volumétrique (200) du type soufflet comprenant au moins une chambre (211, 212, 213, 214) qui définit un volume variable, le capteur de débit volumétrique (200) étant configuré pour alternativement admettre du gaz dans la chambre (211, 212, 213, 214) et ensuite à nouveau expulser le gaz de la chambre (211, 212, 213, 214), l'admission et l'expulsion alternées provoquant des oscillations du flux de gaz (G);
   un capteur de flux thermique (100) configuré pour être exposé au flux de gaz (G); et **caractérisé en ce que** un dispositif de commande (500) configuré pour exécuter le procédé suivant:

   détecter un signal d'oscillation sur la base de signaux provenant du capteur de flux thermique (100), le signal d'oscillation étant indicatif des oscillations du flux de gaz (G); et
   déterminer le débit volumétrique ($dV/dt$) sur la base du signal d'oscillation.

11. Le capteur de gaz selon la revendication 10, dans lequel le dispositif de commande (500) est configuré pour exécuter le procédé suivant pour déterminer un paramètre de combustion ($H_\rho$; $I_W$; $N_M$) du gaz:

   calculer un paramètre de transfert de chaleur ($\lambda_0$) et un paramètre de capacité thermique ($c_p\rho_0$) du gaz provenant d'une sortie du capteur de débit thermique (100) et du débit volumétrique ($dV/dt$); et
   calculer un paramètre de combustion ($H_\rho$; $I_W$; $N_M$) du gaz à partir du paramètre de transfert de chaleur ($\lambda_0$) et du paramètre de capacité thermique ($c_p\rho_0$).

12. Le capteur de gaz selon la revendication 11,
   dans lequel le capteur de flux thermique (100) comprend un élément chauffant (101), un premier capteur de température (102) en amont de l'élément chauffant (101), et un second capteur de température (103) en aval de l'élément chauffant (101), et dans lequel le dispositif de commande (500) est configuré pour effectuer les étapes suivantes:

   faire fonctionner le capteur de flux thermique (100) pour déterminer une première valeur de température ($T_1$) au niveau du premier capteur de température (102) et une seconde valeur de température ($T_2$) au niveau du second capteur de température (103) sous l'action de l'élément chauffant (101);

calculer le paramètre de transfert de chaleur ($\lambda_0$) et le paramètre de débit massique ($\phi$) du gaz en tenant compte des dites première et seconde valeurs de température ($T_1$, $T_2$); et

calculer le paramètre de capacité thermique ($c_p\rho_0$) à partir du paramètre de débit massique ($\phi$), du débit volumétrique ($dV/dt$), et du paramètre de transfert de chaleur ($\lambda_0$)

13. Un procédé de rétrofit d'un compteur à gaz de type soufflet existant (200), dans lequel le compteur à gaz (200) comprend au moins une chambre (211, 212, 213, 214) qui définit un volume variable, le compteur à gaz (200) étant configuré pour alternativement admettre du gaz dans la chambre (211, 212, 213, 214) et pour ensuite à nouveau expulser le gaz de la chambre (211, 212, 213, 214), l'admission et l'expulsion alternées provoquant des oscillations du flux de gaz (G);

le procédé comprenant le rétrofit du compteur à gaz (200) avec un capteur de flux thermique (100) configuré pour être exposé au flux de gaz (G) et avec un dispositif de commande (500) qui est configuré pour effectuer le procédé suivant:

détecter un signal d'oscillation sur la base de signaux provenant du capteur de flux thermique (100), le signal d'oscillation étant indicatif des oscillations du flux de gaz (G); et

déterminer le débit volumétrique ($dV/dt$) sur la base du signal d'oscillation.

14. Un programme informatique comprenant un code de programme informatique qui, lorsqu'il est exécuté dans un dispositif de commande (500) d'un capteur de gaz selon l'une quelconque des revendications 10 à 12, amène le dispositif de commande (500) à exécuter un procédé selon l'une quelconque de revendications 1 à 9.

Hₑ

10

#cycles

T1, T2

500

T          P

11

300    400    100

200

G                                                    G

**FIG. 1**

100

107   101  108

G

T1        T2

104   102   105   106   103   104

**FIG. 2**

200

202 — G ↓   G ↑ — 203

204

201

211   212   213 ⟹ 214

207   206   205

**FIG. 3**

208

209

Flow [a.u.]

701

0   2   4   6   8   10   12
time [sec]

**FIG. 4**

FIG. 6

FIG. 5

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10122039 A1
- DE 4118781 A1
- WO 2015075278 A
- US 7377152 B2
- EP 2086271 A1
- EP 2574918 A1

- EP 0554095 A
- US 2013199290 A
- WO 0198736 A1
- EP 2527779 A1
- WO 2012021999 A1
- US 7188519 B2

**Non-patent literature cited in the description**

- *Report on gas composition range in Europe,* 01 October 2015, http://www.ingas-eu.org/docs/DB0.1.pdf